# EUROPEAN PATENT SPECIFICATION

(11) **EP 4 255 388 B1**
(45) Date of publication and mention of the grant of the patent: **24.12.2025**
(21) Application number: 21802011.3
(22) Date of filing: 25.10.2021
(51) Int. Cl.: A61K 9/00, A61K 9/16, A61K 9/20, A61K 9/50, A61K 31/80, A61K 45/06, A61P 1/00

(54) **MULTI-LAYERED PARTICLE COMPRISING SIMETHICONE**
MEHRSCHICHTIGES PARTIKEL MIT SIMETHICON
PARTICULE MULTICOUCHE COMPRENANT DE LA SIMÉTHICONE

(30) Priority: 03.12.2020 US 202063120791 P; 09.12.2020 SE 2051434
(43) Date of publication of application: 11.10.2023
(73) Proprietor: Kenvue Brands LLC, Summit, NJ 07901 (US)
(72) Inventor: PANDEY, Anurag, Fort Washington, Pennsylvania 19034 (US); DAVE, Vipul, Bridgewater, New Jersey 08807 (US)
(74) Representative: Carpmaels & Ransford LLP
(86) International application number: PCT/IB2021/059841
(87) International publication number: WO 2022/118107

(56) References cited:
- US-A1- 2009 068 263
- US-A1- 2019 099 379

## Description

### FIELD OF THE INVENTION

The present invention is directed to an orally disintegrable multi-layered particle comprising a hydrophobic core containing simethicone, a hydrophilic surfactant layer, and a hydrophilic disintegrant layer; wherein said hydrophilic surfactant layer is located between the hydrophobic core and the hydrophilic disintegrant layer; and orally disintegrable solid dosage forms comprising said multi-layered particle, and related processes.

### BACKGROUND OF THE INVENTION

The present invention relates to novel simethicone multi-layered particles and methods for making such multi-layered particles.

Simethicone has been used to treat intestinal discomfort, pressure, fullness, and bloating. It is typically administered in a liquid or solid form either alone or in combination with antacids or antidiarrheals, such as loperamide.

Simethicone can be administered orally as a liquid preparation or as solid form for example capsules, chewable, swallowable or orally disintegrating tablets. The advantage of tablets over liquids is the ease of portability. The advantages of orally disintegrating tablets over chewable or swallowable tablets include the ease of administration, particularly for geriatric or pediatric patients, and the rapid release of the active ingredient in the oral cavity.

Typically, to incorporate simethicone into a solid formulation, it must first be adsorbed onto a suitable porous carrier or substrate. Without an appropriate carrier, the highly hydrophobic simethicone forms lumps that are not water soluble or disintegrable. There have been several inventions related to this problem using various substrate materials from polysaccharides to inorganic materials such as calcium phosphates, Calcium carbonate or metalo-silicates. A limitation of the polysaccharide approach is limited loading capacity i.e. a stable concentration of simethicone adsorbed onto the porous substrate resides in the range of 20-25% which implies a simethicone/adsorbate dose of 500-625mg for a 125 mg dose of simethicone. A drawback of the inorganic substrates is their insolubility and gritty mouthfeel. Thus, these approaches to generating solid phase simethicone powder or granules are not suitable for certain delivery formats such as orally disintegrating tablets (ODTs) or orally dispersible granules (ODGs).

Particles loaded with simethicone are highly hydrophobic. Historically, in order to overcome this hydrophobicity and obtain rapidly disintegrating tablets, superdisintegrants were mixed with the simethicone blend inside the particles. Another technique to lower the hydrophobicity of the simethicone loaded particles was simply to lower the simethicone loading, thus resulting in larger tablets and longer disintegration times.

Finally, particles loaded with simethicone tend to be tacky and adhere to each other. This issue was addressed by coating the particles. The free-flowing properties thus imparted to the particles are highly desirable to reduce the risk of clogging and/or non-uniformity, and should be maintained in newly developed simethicone particles.

It is therefore desirable to propose a simethicone particle that would address the limitations of the existing approaches by improving the simethicone powders available for ODT and ODG manufacturing. It is also desirable that the proposed simethicone powders remain free flowing.

The benefit of the novel simethicone multi-layered particle is that it addresses the limitations of the existing approaches to producing free flowing simethicone powders. Additional benefits of the multi-layered particle are the high simethicone to porous substrate ratio allowing dosage forms containing a higher load of simethicone or, alternatively, smaller dosage forms having similar simethicone content to the larger prior art dosage forms. Another advantage of the dosage forms obtained with the novel multi- layer particles is their disintegration in less than 60 seconds in the oral cavity.
US 2019/099379 is directed to microencapsulated simethicone particles containing simethicone and a water soluble coating, wherein the simethicone is about 50% by weight of the particle.

### SUMMARY OF THE INVENTION

The invention relates to the development of new multi-layered Simethicone particles. Such multi-layered particles allow for new solid dosage forms that have improved properties compared to present solid dosage forms including improved dispersion time in the oral cavity, a higher load of simethicone and improved blending with other material. The newly developed multi-layered Simethicone particle retains a free-flowing profile for ease of processability.

In a first aspect the present invention is directed to a multi-layered particle, orally disintegrating, comprising at least:
a) a hydrophobic core containing simethicone absorbed on a porous substrate of Calcium phosphate, Calcium carbonate, amorphous silicate, or a mixture thereof,
b) a hydrophilic surfactant layer comprising a polysorbate surfactant, and
c) a hydrophilic disintegrant layer comprising
   i) a hydrophilic binder such as a cellulose derivative or a polyvinyl alcohol-polyethylene glycol copolymer, or a mixture thereof, and
   ii) a disintegrant such as a cross-linked cellulose polymer or cross-linked povidone or sodium starch glycolate, or a mixture thereof,
wherein said hydrophilic surfactant layer is located between the hydrophobic core and the hydrophilic disintegrant layer.

It has surprisingly been found that a hydrophilic surfactant layer between the hydrophobic core and the hydrophilic disintegrant layer improves the disintegration of the multi-layered particle in the oral cavity. Formulation of the particle with both this hydrophilic surfactant layer and the hydrophilic disintegrant layer allows for the formation of free-flowing particles.

Improved disintegration of the multi-layered particle allows for an increase in the simethicone load of said particle. Thus, an increase of the hydrophobicity of the core is proposed without significantly affecting the disintegration time.

In a second aspect the present invention also includes a solid dosage form comprising
a) from 30% to 50%, in weight, of a plurality of said multi-layered particles, and
b) from 50% to 70%, in weight, of a solid excipient phase comprising microcrystalline cellulose and polyols.

In another aspect, the present invention is directed to a method of preparing multi layered particle comprising:
a) blending simethicone with a porous substrate of Calcium phosphate, Calcium carbonate, amorphous silicate, or a mixture thereof, to achieve a hydrophobic particle, and ensure complete adsorption and uniform distribution,
b) wet coating said hydrophobic particle by spraying a solution comprising a polysorbate surfactant,
c) adding a hydrophilic binder such as a cellulose derivative or a polyvinyl alcohol-polyethylene glycol copolymer, or a mixture thereof, and a super disintegrant such as a cross linked cellulose polymer or cross-linked povidone or sodium starch glycolate, or a mixture thereof, to coat the surface of the particles.

Finally, the present invention also includes the said multi layered particle for use in the treatment of disease or disorder in the gastrointestinal tract, for example diarrhea or digestive conditions.

### DETAILED DESCRIPTION OF THE INVENTION

Unless provided otherwise, all % are expressed in weight %.

By multi layered particle it is meant an entity comprising a core, the surface of said core being covered, or wrapped, by at least two layers, namely a hydrophilic surfactant layer and a hydrophilic disintegrant layer. The hydrophilic surfactant layer is covering, or wrapping, the core surface. The hydrophilic disintegrant layer is covering, or wrapping, the hydrophilic surfactant layer surface.

In other words, the said layers may be considered as coating layers. So, in this wording, the hydrophilic surfactant layer is coating the core; and the hydrophilic disintegrant layer is coating said hydrophilic surfactant layer.

The invention relates to a multi-layered particle, orally disintegrating, comprising at least:
a) a hydrophobic core containing simethicone absorbed on a porous substrate of Calcium phosphate, Calcium carbonate, amorphous silicate, or a mixture thereof,
b) a hydrophilic surfactant layer comprising a polysorbate surfactant, and
c) a hydrophilic disintegrant layer comprising
   i) a hydrophilic binder such as a cellulose derivative or a polyvinyl alcohol-polyethylene glycol copolymer, or a mixture thereof, and
   ii) a disintegrant such as a cross linked cellulose polymer or cross-linked povidone or sodium starch glycolate, or a mixture thereof,
wherein said hydrophilic surfactant layer is located between the hydrophobic core and the hydrophilic disintegrant layer.

The multi-layered particles may have a diameter equal or smaller than 0.7 mm, such as equal or smaller than 0.5 mm.

The porous substrate may be Magnesium Aluminosilicate.

The simethicone may account for at least 25% of the multi-layered particle weight, such as at least 30%, or at least 35% of the multi-layered particle weight. Simethicone may also be present in an amount less than 70% of the multi-layered particle weight, preferably less than 65%, such as 60%, or even 55% of the multi-layered particle weight. In other configurations simethicone may be present in an amount of from 25% to 70% of the multi-layered particle weight, such as from 25% to 60%, or such as from 30% to 70%, or even preferably from 30% to 60%, such as from 35% to 55% (wt%) of the multi-layered particle weight.

The multi-layered particle according to the invention may have a hydrophilic surfactant layer comprising more than 90% of polysorbate surfactant.

Said hydrophilic surfactant layer may comprise more than 95%, or 98%, or 99%, or even 99.5% of polysorbate surfactant. In a preferred formulation the hydrophilic surfactant layer may comprise 100% of polysorbate surfactant.

Preferably the polysorbate surfactant may be Polyoxyethylene (20) sorbitan monolaurate.

The hydrophilic binder may be a Hydroxypropylcellulose.

The disintegrant may be a cross linked Sodium Carboxymethylcellulose.

The disintegrant may also contain fume silica.

In another embodiment the disintegrant may contain cross linked Sodium Carboxymethylcellulose and fumed silica.

Solid dosage form according to the present invention may comprise
a) from 30% to 50%, in weight, of a plurality of multi-layered particles according to the invention, and
b) from 50% to 70%, in weight, of a solid excipient phase comprising microcrystalline cellulose and polyols.

Preferably the solid excipient phase may comprise a blend of Microcrystalline Cellulose, Colloidal Silicon Dioxide, Mannitol, Fructose and Crospovidone.

The solid dosage form may be an orally disintegrating dosage, such as a lozenge, an orally disintegrating tablet, orally disintegrating granules, or chewable tablet for example.

The solid dosage form of the present invention may be an instantly disintegrating form.

More specifically, the solid dosage form of the present invention may be an orally disintegrating tablet.

The solid dosage form may have simethicone present in an amount from 30 mg to 200 mg, preferably from 50mg to 180mg, even more preferably from 100 mg to 150 mg.

The solid dosage form may have simethicone present in an amount greater than or equal to 30 mg, 40 mg, 50 mg, 60mg, 70 mg, 80 mg, 90 mg or 100mg.

The solid dosage form may have simethicone present in an amount less than or equal to 200 mg, 190 mg, 180 mg, 170 mg, 160 mg or 150mg.

The solid dosage form may have simethicone present in an amount from 30 mg to 180 mg, or from 50 mg to 200 mg, or from 50 mg to 180 mg, or from 50 mg to 150 mg, or from 70 mg to 200 mg, or from 70 mg to 180 mg, or from 70 mg to 150 mg, or from 80 mg to 200 mg, or from 80 mg to 180 mg, or from 80 mg to 150 mg.

Alternatively, the solid dosage form may have simethicone accounting for at least 10% of the solid dosage form weight, preferably 15% to 25% (wt%), more preferably 17% to 23% (wt%).

The solid dosage form may have a simethicone to porous substrate weight ratio, in the hydrophobic core, of about from 1 : 3, up to 3 : 1, preferably from about from 1 : 2.5, up to 2.5 : 1, even more preferably of about from 1 : 2, up to 2 : 1.

For the sake of clarity, by a weight ratio from 1:2 up to 2:1 of simethicone to porous substrate it is meant that the mass of simethicone may be half of the mass of porous substrate, up to a mass of simethicone twice the mass of porous substrate.

The solid dosage form may have the porous substrate of Calcium phosphate, calcium carbonate, amorphous silicate, or a mixture thereof, account for at least 10% of the solid dosage form weight, preferably 10% to 25% (wt%).

The solid dosage form may have the hydrophilic surfactant layer account for 0.1% to 1% of the solid dosage form weight, preferably 0.4% to 0.7% (wt%).

The solid dosage form may have the hydrophilic binder such as a cellulose derivative or a polyvinyl alcohol-polyethylene glycol copolymer account 1% to 5% of the solid dosage form weight, preferably 2% to 4% (wt%).

The solid dosage form may have the disintegrant account for 1% to 7% of the solid dosage form weight, preferably 1 % to 6 % (wt%), 1 % to 5 % (wt%), preferably 1.5% to 4% (wt%).

The solid dosage form may have cross linked cellulose polymer account for 0.5% to 3% of the solid dosage form weight, preferably 1% to 2.5% (wt%), more preferably 1.5% to 2.5% (wt%), and wherein the cross linked cellulose polymer is part of the disintegrant.

The solid dosage form may have a solid excipient phase comprising microcrystalline cellulose and polyols accounting for at least 40% of the solid dosage form weight, preferably 55% to 65% (wt%).

The solid dosage form may also contain other components such as lubricant, glidant, sweeteners, flavoring agents/flavors, coloring agents, or fillers.

Suitable lubricants include long chain fatty acids and their salts, such as magnesium stearate and stearic acid, talc, glycerides waxes, and mixtures thereof.

Suitable glidant is colloidal silicon dioxide.

Examples of sweeteners include, synthetic or natural sugars; artificial sweeteners such as saccharin, sodium saccharin, aspartame, neotame, acesulfame, thaumatin, glycyrrhizin, sucralose, cyclamate, dihydrochalcone, alitame, miraculin and monellin; sugar alcohols such as sorbitol, mannitol, glycerol, lactitol, maltitol, and xylitol; sugars extracted from sugar cane and sugar beet (sucrose), dextrose (also called glucose), fructose (also called laevulose), and lactose (also called milk sugar); isomalt, stevia, and mixtures thereof.

Examples of flavoring agents/flavors include, fruit and berry flavors such as lime, orange, , lemon, black current, blood orange, cranberry, cloudberry, goji berry, raspberry, strawberry, wild strawberry, sea buckthorn, cherry, melon, kiwi, papaya, pineapple, passion fruit, coconut, and other flavors such as honey, herbs, the, anise, water grass, lemon grass, cooling agent ginger, coffee, eucalyptus, mangostan, peppermint, spearmint, wintergreen, cinnamon, cacao/cocoa, vanilla, liquorice, salt, pepper, chili, menthol, aniseeds, mint or mixtures thereof. The flavoring agents/flavors may be natural extracts as well as synthetic versions.

Examples of coloring agents include lakes and dyes being approved as a food additive.

Examples of fillers that may be used include maltitol, xylitol, sorbitol, mannitol, lactose, dextrose, saccharose or fructose, or any mixture thereof. One example is mannitol.

In one embodiment the solid dosage form may have a disintegration time below or equal to 60 seconds, preferably below or equal to 45 seconds, even preferably below or equal to 30 seconds.

In a preferred embodiment the solid dosage form according to the invention, may be an orally disintegrating tablet comprising
- from 30% to 50%, in weight, of a plurality of multi-layered particles, said particles comprising
   a) a hydrophobic core containing simethicone absorbed on Magnesium Aluminosilicate
   b) a hydrophilic surfactant layer comprising Polyoxyethylene (20) sorbitan monolaurate, and
   c) a hydrophilic disintegrant layer comprising
      i) a hydrophilic binder such as a Hydroxypropylcellulose or a polyvinyl alcohol-polyethylene glycol copolymer, or a mixture thereof, and
      ii) a disintegrant such as a cross linked Sodium Carboxymethylcellulose,
- from 50% to 70%, in weight, of a solid excipient phase comprising microcrystalline cellulose and polyols, and
wherein said hydrophilic surfactant layer is located between the hydrophobic core and the hydrophilic disintegrant layer.

An alternative embodiment of the invention may be a solid dosage form, as an orally disintegrating tablet, comprising a plurality of multi-layered particles, said particles comprising:
a) a hydrophobic core containing 20% to 25% simethicone absorbed on 10% to 25% Magnesium Aluminosilicate
b) 0.4% to 0.6% of a hydrophilic surfactant layer comprising Polyoxyethylene (20) sorbitan monolaurate, and
c) a hydrophilic disintegrant layer comprising
   i) 2% to 4% of a hydrophilic binder such as a Hydroxypropylcellulose or a polyvinyl alcohol-polyethylene glycol copolymer, or a mixture thereof, and
   ii) 1.5% to 2.5% a disintegrant such as a cross linked Sodium Carboxymethylcellulose, and
55% to 65% of a solid excipient phase comprising microcrystalline cellulose and polyols, and wherein said hydrophilic surfactant layer is located between the hydrophobic core and the hydrophilic disintegrant layer, and all % are expressed in weight% of the solid dosage form.

In addition, the solid dosage form may contain at least one additional pharmaceutical ingredient, selected among: famotidine, ranitidine, cimetidine, racecadotril, bismuth subsalicylate, calcium carbonate, sodium bicarbonate, aluminum hydroxide, magnesium hydroxide, magnesium oxide, hyoscine or antispasmodic drugs such as dicyclomine and hyoscyamine.

In a preferred embodiment, the additional pharmaceutical ingredient is Loperamide. The solid dosage form of the invention may contain Loperamide.

In a particular embodiment, the solid dosage form may contain Calcium carbonate as a porous substrate in the hydrophobic core. In that configuration, Calcium carbonate has the role of both porous substrate and additional pharmaceutical ingredient.

Alternatively, Calcium carbonate may be present as an additional pharmaceutical ingredient and a porous substrate different from Calcium carbonate may be present as a porous substrate in the hydrophobic core.

The additional pharmaceutical ingredient may be blended to the hydrophilic disintegrant layer.

Alternatively, the additional pharmaceutical ingredient may be blended to the solid excipient phase.

In yet another embodiment the additional pharmaceutical ingredient may be blended in both the hydrophilic disintegrant layer and the solid excipient phase.

In another embodiment, the present invention relates to a process for the production of an orally disintegrable multi layered particle comprising:
a) blending simethicone with a porous substrate of Calcium phosphate, Calcium carbonate, amorphous silicate, or a mixture thereof, to achieve a hydrophobic particle, and ensure complete adsorption and uniform distribution,
b) wet coating said hydrophobic particle by spraying a solution comprising a polysorbate surfactant,
c) adding a hydrophilic binder such as a cellulose derivative or a polyvinyl alcohol-polyethylene glycol copolymer, or a mixture thereof, and a super disintegrant such as a cross linked cellulose polymer or cross linked povidone or sodium starch glycolate, or a mixture thereof, to coat the surface of the particles.

Further, the invention also relates to a process for the production of a solid dosage form comprising:
a) blending in a mixer the multi layered particles and a solid excipient phase comprising microcrystalline cellulose and polyols, and
b) compressing the obtained blend into a solid dosage from.

Preferably the blend of multi layered particles and a solid excipient may be compressed in a pressure range from 2000 to 6000 pounds, preferably 2500 to 5000 pounds, or even preferably 2500 to 4000 pounds.

Preferably the blend of multi layered particles and a solid excipient may be compressed to a hardness range from 0.4 to 3.5 kp, preferably 0.5 to 3 kp, even more preferably 0.8 to 2.5 kp.

The invention also relates to the use of the multi layered particle for the manufacture of a solid dosage form with oral disintegrating properties.

In a final aspect the invention relates to the multi layered particle for use in the treatment of disease or disorder in the gastrointestinal tract, for example diarrhea or digestive conditions such as dehydration, abdominal pain, bloating, nausea, flatulence, cramping or Irritable Bowel Syndrome.

In other words, the invention relates to the use of the multi layered particle for the manufacturing of a solid dosage form for the treatment of disease or disorder in the gastrointestinal tract, for example diarrhea or digestive conditions such as dehydration, abdominal pain, bloating, nausea, flatulence, cramping or Irritable Bowel Syndrome.

The invention also relates to the multi layered particle, or the solid dosage form, disclosed above, for use in a method of treatment of a subject suffering from a disease or disorder in the gastro intestinal tract, for example diarrhea or digestive conditions such as dehydration, abdominal pain, bloating, nausea, flatulence, cramping or Irritable Bowel Syndrome.

### EXPERIMENTAL PART

The hardness test (or crushing hardness) is based on hardness of the dosage form measured perpendicular to the cross-section at the belly band using a modified Model 6d, Pharmatron hardness tester fitted with a 50g force load cell. Hardness is measured in kp or kilopond.

Friability is measured using the USP 24 NF 29 Tablet Friability (Section 1216) with the modification of using 3 tablets for 15 rotations or 3 tablets for 100 revolutions (unless otherwise noted) instead of 10 tablets for 100 rotations.

DT means Disintegration time. DT was measured using 900mL of deionized water at 37°C, based on the United States Pharmacopeia (USP 24 NF 29) disintegration test method for the specific medicinal substance or substances.

Neusilin US2 was purchased from the Fuji Chemical Corporation, Burlington, NJ, USA.

Fujicalin was purchased from the Fuji Chemical Corporation, Burlington, NJ, USA.

Simethicone USP (Q7-2243 LVA) was purchased from the Dow Chemical Corporation, Midland, MI, USA.

Polysorbate 20 was purchased from the BASF Corporation, Florham Park, NJ, USA.

Kollicoat Protect was purchased from the BASF Corporation, Florham Park, NJ, USA.

HPC Klucel EFX was purchased from the Ashland Corporation, Bridgewater, NJ, USA.

Ac-di-sol was purchased from the Dupont Pharma Corporation, Midland, MI, USA.

ProSolv ODT was purchased from the JRS Pharma Corporation, Patterson, NY, USA.

ProSolv ODT G2 was purchased from the JRS Pharma Corporation, Patterson, NY, USA.

Precipitated Calcium Carbonate (Vicality Extra Light Precipitated Calcium Carbonate) was purchased from Minerals Technologies Inc., Adams, MA, USA.

### EXAMPLE 1 (COMPARATIVE EXAMPLE):

The following example is outside the scope of the present invention because it does not contain a hydrophilic surfactant layer. All the disintegration times are over 2 minutes.

In a Hobart mixer 50 g Neusilin US2 is weighed out and while mixing at low speed, 100 g of Simethicone, USP is added, mixing is continued for 10 minutes to ensure complete adsorption and uniform distribution. While mixing slowly add 20 g of Kollicoat Protect and 10 g of Ac-di-sol is slowly added while mixing to coat the wet Neusilin particles surface with Kollicoat Protect and Ac-di sol. 60 g of coated Simethicone loaded Neusilin powder is passed through a 30 mesh and blended with 60 g of ProSolv ODT in a turbula mixer. 530 mg tablets were compressed using a Natoli tablet hand press.

Table 1 shows the compression studies and dissolution times for tablets according to example 1.

**Table 1**

| Pressure (pound) | Weight (mg) | Thickness (mm) | Hardness (kp) | Friability (%, in weight, of mass loss) | | DT(min) |
|---|---|---|---|---|---|---|
| | | | | 15 Revolutions | 100 Revolutions | |
| 2000 | 534.5 | 3.94 | 0.35, 0.36 | 0.43 | Fail | 2.22, 2.21 |
| 3900 | 511.4 | 3.43 | 0.59, 0.61 | 0.38 | 1.16 | 2.38, 2.36 |
| 5800 | 518.2 | 3.46 | 0.59, 0.57 | 0.19 | 0.51 | 3.02, 3.04 |

For friability test, "Fail" means that the weight loss is above 2% or that the tablet breaks down in small pieces.

For pressure, 1 pound force is equivalent to about 4.448N, so a 2000 pounds pressure is equivalent to about 8.9 kN.

### EXAMPLE 2:

In a Hobart mixer 50 g Neusilin US2 is weighed out and while mixing at low speed, 100 g of Simethicone, USP is added, mixing is continued for 5 minutes to ensure complete adsorption and uniform distribution. While mixing a 25 mL of 12% solution of Polysorbate 20 in water is sprayed on over next 2-5 minutes and mixing is continued for an additional 5 minutes. To the wet mass 15 g of Kollicoat Protect and 10 g of Ac-di-sol is slowly added while mixing rapidly to coat the wet Neusilin particles surface with Kollicoat Protect and Ac-di sol. The wet mass contains small amount of aggregates and is dried overnight in a pan at 50°C. After drying, 40 g of the dried Simethicone loaded Neusilin is passed through a #35 mesh and blended with 60 g of ProSolv ODT in a turbula mixer. 530 mg tablets were compressed using a Natoli tablet hand press.

Table 2 shows the compression studies and dissolution times for tablets according to example 2.

**Table 2**

| Pressure (pound) | Weight (mg) | Thickness (mm) | Hardness (kp) | Friability (%, in weight, of mass loss) | | DT (min) |
|---|---|---|---|---|---|---|
| | | | | 15 Revolutions | 100 Revolutions | |
| 2500 | 537.8 | 3.85 | 0.45, 0.55 | Fail | Fail | 0.22, 0.24 |
| 4000 | 545.8 | 3.57 | 1.42, 1.33, 1.48 | 0.12 | 1.78 | 0.25, 0.23 |
| 5000 | 534.8 | 3.39 | 2.07, 2.15, 1.98 | 0.25 | 0.81 | 0.31, 0.33 |

### EXAMPLE 3:

In a Hobart mixer 50 g Neusilin US2 is weighed out and while mixing at low speed, 100 g of Simethicone, USP is added, mixing is continued for 5 minutes to ensure complete adsorption and uniform distribution. While mixing a 25 mL of 12% solution of Polysorbate 20 in water is sprayed on over next 2 minutes and mixing is continued for an additional 5 minutes. To the wet mass 15 g of Kollicoat Protect and 10 g of Ac-di-sol is slowly added while mixing rapidly to coat the wet Neusilin particles surface with Kollicoat Protect and Ac-di sol. The wet mass contains small amount of aggregates and is dried overnight in a pan at 50°C. After drying, 40 g of the dried Simethicone loaded Neusilin is passed through a 30 mesh and blended with 60 g of ProSolv ODT G2 in a turbula mixer. 530 mg tablets were made using a Natoli tablet hand press.

Table 3 shows the compression studies and dissolution times for tablets according to example 3.

**Table 3**

| Pressure (pound) | Weight (mg) | Thickness (mm) | Hardness (kp) | % Friability (%, in weight, of mass loss) | | DT(min) |
|---|---|---|---|---|---|---|
| | | | | 15 Revolutions | 100 Revolutions | |
| 2500 | 530.6 | 4.06 | 0.48, 0.40 | 0.82 | Fail | 0.16, 0.16 |
| 4000 | 533.5 | 3.69 | 1.29, 1.33 | 0.25 | 1.64 | 0.16, 0.18 |
| 5000 | 533.2 | 3.54 | 2.05, 1.99 | 0.06 | 1.84 | 0.31, 0.26 |
| 6000 | 537.5 | 3.46 | 2.33, 2.90 | 0.06 | 0.18 | 0.51, 0.53 |

### EXAMPLE 4:

In a Hobart mixer 75 g Neusilin US2 is weighed out and while mixing at low speed, 150 g of Simethicone, USP is added, mixing is continued for 5 minutes to ensure complete adsorption and uniform distribution. While mixing a 33g of 12% solution of Polysorbate 20 in water is sprayed on over next 2 minutes and mixing is continued for an additional 5 minutes. To the wet mass 22.5 g of HPC Klucel EFX and 15 g of Ac-di-sol is slowly added while mixing rapidly to coat the wet Neusilin particles surface with HPC Klucel EFX and Ac-di sol. The wet mass contains small amount of aggregates and is dried overnight in a pan at 50°C. After drying, 40 g of the dried Simethicone loaded Neusilin is passed through a #35 mesh and blended with 60 g of ProSolv ODT G2 in a turbula mixer. 530 mg tablets were made using a Natoli tablet hand press.

Table 4 shows the compression studies and dissolution times for tablets according to example 4.

**Table 4**

| Pressure (pound) | Weight (mg) | Thickness (mm) | Hardness (kp) | % Friability (%, in weight, of mass loss) | | DT(min) |
|---|---|---|---|---|---|---|
| | | | | 15 Revolutions | 100 Revolutions | |
| 2500 | 606.3 | 4.30 | 0.96, 0.94 | 0.06 | 1.62 | 0.15, 0.13 |
| 4000 | 617.0 | 4.07 | 1.73, 1.83 | 0.21 | 0.32 | 0.33, 0.27 |
| 5000 | 619.6 | 4.08 | 1.90, 1.73 | 0.0 | 0.22 | 0.33, 0.33 |
| 6000 | 625.9 | 4.12 | 1.91, 1.84 | 0.10 | 0.21 | 0.33, 0.34 |

### EXAMPLE 5:

In a Hobart mixer 110g Fujicalin is weighed out and while mixing at low speed, 70 g of Simethicone, USP is added, mixing is continued for 5 minutes to ensure complete adsorption and uniform distribution. While mixing a 16g of 12% solution of Polysorbate 20 in water is sprayed on over next 2 minutes and mixing is continued for an additional 5 minutes. To the wet mass 10 g of HPC Klucel EFX and 10 g of Ac-di-sol is slowly added while mixing rapidly to coat the wet Fujicalin particles surface with HPC EFX and Ac-di sol. The wet mass contains small amount of aggregates and were dried overnight in a pan at 50°C. After drying Simethicone loaded Fujicalin particles overnight 1.5 g of Cab-O-Sil was blended and passed through a #35 mesh. 40 g of this material was blended with 60 g of ProSolv ODT G2 in a turbula mixer. 875 mg tablets were made using a Natoli tablet hand press.

Table 5 shows the compression studies and dissolution times for tablets according to example 5.

**Table 5**

| Pressure (pound) | Weight (mg) | Thickness (mm) | Hardness (kp) | % Friability (%, in weight, of mass loss) | | DT(min) |
|---|---|---|---|---|---|---|
| | | | | 15 Revolutions | 100 Revolutions | |
| 2500 | 912.9 | 6.12 | 0.94, 0.96 | 0.29 | 6.70 | 0.24, 0.22 |
| 4000 | 876.7 | 5.36 | 2.35, 2.49 | 0.26 | 1.45 | 0.24, 0.26 |
| 5000 | 895.4 | 5.23 | 3.85,3.69 | 0.06 | 1.14 | 1.14, 1.04 |

### EXAMPLE 6:

In a Hobart mixer 120 g of Vicality Extra Light Precipitated Calcium Carbonate is weighed out and while mixing at low speed, 75 g of Simethicone, USP is added, mixing is continued for 5 minutes, while mixing 35 g Neusilin US2 is added followed by addition of 75 g of Simethicone, USP. Mixing is continued at low speed for an additional 5 min to ensure complete adsorption and uniform distribution of Simethicone. While mixing a 33g of 12% solution of Polysorbate 20 in water is sprayed on over next 2 minutes and mixing is continued for an additional 5 minutes. To the wet mass 22.5 g of HPC Klucel EFX and 15 g of Ac-di-sol is slowly added while mixing rapidly to coat the wet particles surface with HPC Klucel EFX and Ac-di sol. The wet mass contains small amount of aggregates and is dried overnight in a pan at 50°C. After drying, 40 g of the dried Simethicone loaded particles are passed through a #30 mesh and blended with 60 g of ProSolv ODT G2 in a turbula mixer. 690 mg tablets were made using a Natoli tablet hand press.

Table 6 shows the compression studies and dissolution times for tablets according to example 6.

**Table 6**

| Pressure (pound) | Weight (mg) | Thickness (mm) | Hardness (kp) | % Friability (%, in weight, of mass loss) | | DT(min) |
|---|---|---|---|---|---|---|
| | | | | 15 Revolutions | 100 Revolutions | |
| 2000 | 703.4 | 5.23 | 0.89, 0.86 | 0.45 | 3.74 | 0.14, 0.09 |
| 4000 | 686.7 | 5.06 | 1.88, 1.69 | 0.16 | 1.09 | 0.22, 0.23 |
| 5000 | 691.2 | 4.83 | 2.09, 2.38 | 0.16 | 0.51 | 0.36, 0.43 |

## Claims

1. Multi-layered particle, orally disintegrating, comprising at least:
a) a hydrophobic core containing simethicone absorbed on a porous substrate of Calcium phosphate, Calcium carbonate, amorphous silicate, or a mixture thereof,
b) a hydrophilic surfactant layer comprising a polysorbate surfactant, and
c) a hydrophilic disintegrant layer comprising
i) a hydrophilic binder such as a cellulose derivative or a polyvinyl alcohol-polyethylene glycol copolymer, or a mixture thereof, and
ii) a disintegrant such as a cross linked cellulose polymer or cross linked povidone or sodium starch glycolate, or a mixture thereof,
wherein said hydrophilic surfactant layer is located between the hydrophobic core and the hydrophilic disintegrant layer.

2. Multi-layered particle according to claim 1, wherein the multi-layered particles have a diameter equal or smaller than 0,7 mm, such as equal or smaller than 0,5 mm.

3. Multi-layered particle according to any of the preceding claims, wherein the porous substrate is a Magnesium Aluminosilicate.

4. Multi-layered particle according to any of the preceding claims, wherein the hydrophilic surfactant layer comprises more than 90%, in weight %, of polysorbate surfactant.

5. Multi-layered particle according to any of the preceding claims, wherein the polysorbate surfactant is Polyoxyethylene (20) sorbitan monolaurate.

6. Multi-layered particle according to any of the preceding claims, wherein the hydrophilic binder is a Hydroxypropylcellulose.

7. Multi-layered particle according to any of the preceding claims, wherein the disintegrant is a cross linked Sodium Carboxymethylcellulose.

8. Solid dosage form comprising
a) from 30% to 50%, in weight, of a plurality of multi-layered particles according to claims 1 to 7, and
b) from 50% to 70%, in weight, of a solid excipient phase comprising microcrystalline cellulose and polyols.

9. Solid dosage form according to claim 8, wherein the dosage form is an orally disintegrating dosage, such as a lozenge, an orally disintegrating tablet, orally disintegrating granules, or chewable tablet.

10. Solid dosage form according to claims 8 or 9, wherein the simethicone is present in an amount from 30 mg to 200 mg.

11. Solid dosage form according to claims 8 to 10, containing at least one additional pharmaceutical ingredient, selected among: famotidine, ranitidine, cimetidine, racecadotril, bismuth subsalicylate, calcium carbonate, sodium bicarbonate, aluminum hydroxide, magnesium hydroxide, magnesium oxide, hyoscine or antispasmodic drugs such as dicyclomine and hyoscyamine.

12. Process for the production of an orally disintegrable multi layered particle, comprising:
a) blending simethicone with a porous substrate of Calcium phosphate, Calcium carbonate, amorphous silicate, or a mixture thereof, to achieve a hydrophobic particle, and ensure complete adsorption and uniform distribution,
b) wet coating said hydrophobic particle by spraying a solution comprising a polysorbate surfactant,
c) adding a hydrophilic binder such as a cellulose derivative or a polyvinyl alcohol-polyethylene glycol copolymer, or a mixture thereof, and a super disintegrant such as a cross linked cellulose polymer or cross-linked povidone or sodium starch glycolate, or a mixture thereof, to coat the surface of the particles.

13. Use of multi layered particle according to claims 1 to 7, for the manufacture of a solid dosage form with oral disintegrating properties.

14. The multi layered particle according to claims 1 to 7, for use in the treatment of disease or disorder in the gastrointestinal tract, for example diarrhea or digestive conditions such as dehydration, abdominal pain, bloating, nausea, flatulence, cramping or Irritable Bowel Syndrome.

## Patentansprüche

1. Mehrschichtiges, oral zerfallendes Partikel, umfassend mindestens:
a) einen hydrophoben Kern, der Simeticon enthält, das auf einem porösen Substrat aus Calciumphosphat, Calciumcarbonat, amorphem Silikat oder einer Mischung daraus absorbiert ist,
b) eine hydrophile Tensidschicht, umfassend ein Polysorbat-Tensid, und
c) eine hydrophile Sprengmittelschicht, umfassend
i) ein hydrophiles Bindemittel, wie etwa ein Cellulosederivat oder ein Polyvinylalkohol-Polyethylenglykol-Copolymer oder ein Gemisch daraus, und
ii) ein Sprengmittel, wie etwa ein vernetztes Cellulosepolymer oder vernetztes Povidon oder Natriumstärkeglykolat oder ein Gemisch daraus,
wobei die hydrophile Tensidschicht zwischen dem hydrophoben Kern und der hydrophilen Sprengmittelschicht angeordnet ist.

2. Mehrschichtiges Partikel nach Anspruch 1, wobei die mehrschichtigen Partikel einen Durchmesser von höchstens 0,7 mm, insbesondere höchstens 0,5 mm, aufweisen.

3. Mehrschichtiges Partikel nach einem der vorhergehenden Ansprüche, wobei das poröse Substrat ein Magnesiumaluminosilikat ist.

4. Mehrschichtiges Partikel nach einem der vorhergehenden Ansprüche, wobei die hydrophile Tensidschicht zu mehr als 90 Gew.-% Polysorbat-Tensid umfasst.

5. Mehrschichtiges Partikel nach einem der vorhergehenden Ansprüche, wobei das Polysorbat-Tensid Polyoxyethylen(20)-sorbitanmonolaurat ist.

6. Mehrschichtiges Partikel nach einem der vorhergehenden Ansprüche, wobei das hydrophile Bindemittel eine Hydroxypropylcellulose ist.

7. Mehrschichtiges Partikel nach einem der vorhergehenden Ansprüche, wobei das Sprengmittel eine vernetzte Natriumcarboxymethylcellulose ist.

8. Feste Darreichungsform, umfassend
a) zu 30 bis 50 Gew.-% eine Vielzahl mehrschichtiger Partikel nach Anspruch 1 bis 7 sowie
b) zu 50 bis 70 Gew.-% eine feste Trägerphase, umfassend mikrokristalline Cellulose und Polyole.

9. Feste Darreichungsform nach Anspruch 8, wobei die Darreichungsform eine oral zerfallende Darreichungsform ist, wie z. B. eine Lutschtablette, eine im Mund zerfallende Tablette, ein im Mund zerfallendes Granulat oder eine Kautablette.

10. Feste Darreichungsform nach Anspruch 8 oder 9, wobei Simethicon in einer Menge von 30 mg bis 200 mg enthalten ist.

11. Feste Dosierungsform nach Anspruch 8 bis 10, die mindestens einen weiteren pharmazeutischen Bestandteil enthält, ausgewählt aus: Famotidin, Ranitidin, Cimetidin, Racecadotril, Wismutsubsalicylat, Calciumcarbonat, Natriumbicarbonat, Aluminiumhydroxid, Magnesiumhydroxid, Magnesiumoxid, Hyoscin oder krampflösenden Mitteln wie Dicyclomin und Hyoscyamin.

12. Verfahren zum Erzeugen eines oral zerfallenden mehrschichtigen Partikels, umfassend:
a) Mischen von Simeticon mit einem porösen Substrat aus Calciumphosphat, Calciumcarbonat, amorphem Silikat oder Gemischs daraus, um ein hydrophobes Partikel zu erhalten und eine vollständige Adsorption und gleichmäßige Verteilung zu gewährleisten,
b) Nassbeschichten des hydrophoben Partikels durch Aufsprühen einer Lösung, umfassend ein Polysorbat-Tensid,
c) Hinzufügen eines hydrophilen Bindemittels, wie etwa eines Cellulosederivats oder eines Polyvinylalkohol-Polyethylenglykol-Copolymers oder eines Gemischs daraus, und eines Supersprengmittels, wie etwa eines vernetzten Cellulosepolymers oder vernetzten Povidons oder Natriumstärkeglykolats oder einer Mischung davon zur Beschichtung der Partikeloberfläche.

13. Verwendung des mehrschichtigen Partikels nach Anspruch 1 bis 7 zur Fertigung einer festen Darreichungsform mit oral zerfallenden Eigenschaften.

14. Mehrschichtiges Partikel nach Anspruch 1 bis 7 zur Verwendung bei der Behandlung von Erkrankungen oder Störungen des Magen-Darm-Trakts, beispielsweise Durchfall oder Verdauungsbeschwerden wie Dehydration, Bauchschmerzen, Blähungen, Übelkeit, Flatulenz, Krämpfen oder Reizdarmsyndrom.

## Revendications

1. Particule multicouche, à désintégration orale, comprenant au moins :
a) un cœur hydrophobe contenant de la siméthicone absorbée sur un substrat poreux de phosphate de calcium, de carbonate de calcium, de silicate amorphe ou un de leurs mélanges,
b) une couche de tensioactif hydrophile comprenant un tensioactif de polysorbate et
c) une couche hydrophile de désintégrant comprenant
i) un liant hydrophile tel qu'un dérivé de cellulose ou un copolymère de poly(alcool vinylique)-polyéthylène glycol, ou un mélange de ceux-ci, et
ii) un désintégrant tel qu'un polymère de cellulose réticulée ou une povidone réticulée ou un glycolate d'amidon de sodium, ou un mélange de ceux-ci,
dans laquelle ladite couche de tensioactif hydrophile est située entre le cœur hydrophobe et la couche de désintégrant hydrophile.

2. Particule multicouche selon la revendication 1, dans laquelle les particules multicouches ont un diamètre égal ou inférieur à 0,7 mm, tel qu'égal ou inférieur à 0,5 mm.

3. Particule multicouche selon l'une quelconque des revendications précédentes, dans laquelle le substrat poreux est un aluminosilicate de magnésium.

4. Particule multicouche selon l'une quelconque des revendications précédentes, dans laquelle la couche de tensioactif hydrophile comprend plus de 90 %, en % en poids, de tensioactif de polysorbate.

5. Particule multicouche selon l'une quelconque des revendications précédentes, dans laquelle le tensioactif polysorbate est un monolaurate de polyoxyéthylène (20) sorbitane.

6. Particule multicouche selon l'une quelconque des revendications précédentes, dans laquelle le liant hydrophile est une hydroxypropylcellulose.

7. Particule multicouche selon l'une quelconque des revendications précédentes, dans laquelle le désintégrant est une carboxyméthylcellulose sodique réticulée.

8. Forme posologique solide comprenant
a) de 30 % à 50 %, en poids, d'une pluralité de particules multicouches selon les revendications 1 à 7, et
b) de 50 à 70 %, en poids, d'une phase d'excipient solide comprenant une cellulose microcristalline et des polyols.

9. Forme posologique solide selon la revendication 8, dans laquelle la forme posologique est une dose à désintégration orale, telle qu'une pastille, un comprimé à désintégration orale, des granules à désintégration orale ou un comprimé pouvant être mâché.

10. Forme posologique solide selon la revendication 8 ou 9, dans laquelle la siméthicone est présente en une quantité de 30 mg à 200 mg.

11. Forme posologique solide selon les revendications 8 à 10, contenant au moins un ingrédient pharmaceutique supplémentaire, choisi parmi : famotidine, ranitidine, cimétidine, racécadotril, sous-salicylate de bismuth, carbonate de calcium, bicarbonate de sodium, hydroxyde d'aluminium, hydroxyde de magnésium, oxyde de magnésium, hyoscine ou des médicaments antispasmodiques tels que la dicyclomine et l'hyoscyamine.

12. Procédé pour la production d'une particule multicouche à désintégration orale, comprenant :
a) mélange de siméthicone avec un substrat poreux de phosphate de calcium, de carbonate de calcium, de silicate amorphe ou d'un mélange de ceux-ci, pour obtenir une particule hydrophobe, et garantir une adsorption complète et une distribution uniforme,
b) enrobage humide de ladite particule hydrophobe par pulvérisation d'une solution comprenant un tensioactif de polysorbate,
c) ajout d'un liant hydrophile tel qu'un dérivé de cellulose ou un copolymère de poly(alcool vinylique)-polyéthylène glycol ou un mélange de ceux-ci, et d'un superdésintégrant tel qu'un polymère de cellulose réticulée ou une povidone réticulée ou un glycolate d'amidon de sodium, ou un mélange de ceux-ci, pour enrober la surface des particules.

13. Utilisation de particules multicouches selon les revendications 1 à 7, pour la fabrication d'une forme posologique solide ayant des propriétés de désintégration orale.

14. Particule multicouche selon les revendications 1 à 7, destinée à être utilisée dans le traitement d'une maladie ou d'un trouble du tractus gastro-intestinal, par exemple la diarrhée ou des affections digestives telles que la déshydratation, les douleurs abdominales, les ballonnements, la nausée, les flatulences, les crampes ou le syndrome de l'intestin irritable.
